(19) **Europäisches Patentamt**

**European Patent Office**

**Office européen des brevets**

(11) **EP 1 414 560 B1**

(12) **EUROPEAN PATENT SPECIFICATION**

(45) Date of publication and mention
of the grant of the patent:
**09.03.2005 Bulletin 2005/10**

(51) Int Cl.[7]: **B01J 8/02**, B01J 8/04,
B01J 19/24

(21) Application number: **02750407.5**

(22) Date of filing: **02.08.2002**

(86) International application number:
**PCT/US2002/024614**

(87) International publication number:
**WO 2003/013715 (20.02.2003 Gazette 2003/08)**

(54) **RADIAL REACTOR LOADING OF A NONOXIDATIVE DEHYDROGENATION**

BELADEN EINES RADIALREAKTORS MIT EINEM NICHT OXIDATIVEN
DEHYDRIERUNGSKATALYSATOR

CHARGEMENT D'UN REACTEUR RADIAL DE DESHYDROGENATION NON OXYDATIVE

(84) Designated Contracting States:
**AT BE BG CH CY CZ DE DK EE ES FI FR GB GR
IE IT LI LU MC NL PT SE SK TR**

(30) Priority: **07.08.2001 US 923658
08.01.2002 US 41422**

(43) Date of publication of application:
**06.05.2004 Bulletin 2004/19**

(73) Proprietor: **Süd-Chemie, Inc.
Louisville, KY 40232-2370 (US)**

(72) Inventors:
 • **WILLIAMS, David, L.
Louisville, Kentucky 40272 (US)**

 • **KOKICKI, Andrzej
Prospect, Kentucky 40059 (US)**
 • **SMITH, Dennis, J.
Louisville, Kentucky 40299 (US)**
 • **MANKIN, Kyle
Prospect, Kentucky 40059 (US)**

(74) Representative: **Westendorp, Michael, Dr.
Patentanwälte
Splanemann Reitzner
Baronetzky Westendorp
Rumfordstrasse 7
80469 München (DE)**

(56) References cited:
**EP-A- 0 532 325        EP-A- 1 142 631
GB-A- 794 915        US-A- 3 620 685**

**Description**

Background of Invention

1. Field of Invention.

The field of art to which this invention pertains is to the composition of a nonoxidative dehydrogenation catalyst bed contained in a radial reactor, wherein the nonoxidative dehydrogenation catalyst bed includes a nonoxidative dehydrogenation catalyst material layer and a layer of an inert material, wherein the inert layer may contain a potassium compound. More particularly, this invention relates to a nonoxidative dehydrogenation catalyst bed for a radial reactor for nonoxidative dehydrogenation reactions, wherein the catalyst bed is divided into inner and outer ring-shaped layers, wherein catalyst material is placed in the outer layer and inert material, which may contain a potassium compound, is placed in the inner layer.

2. Description of related art.

Radial reactors are utilized for a number of different types of catalytic reactions. For example, radial reactors are utilized in ammonia synthesis plants as disclosed in U.S. Patent Nos. 4,880,603 and 5,250,270.

Various designs for radial reactors and the flow pattern of feed streams within those radial reactors have been disclosed in a number of patents, such as those owned by Ammonia Casale S.A. The reactions that occur in those radial reactors are generally heterogenous synthesis reactions, such as ammonia synthesis or methanol synthesis. The reactors and their catalyst beds are designed to encourage various patterns of flow of the feed stream through the catalyst beds within the radial reactors. These radial reactors are disclosed, for example, in U.S. Patent Nos. 5,756,048, 5,006,316, 4,963,338, 4,952,375, 4,904,453, 4,755,362, 4,769,220, 4,405,562 and 4,372,920.

A common commercial chemical process where radial reactors are utilized is the dehydrogenation of hydrocarbons. The process for the dehydrogenation of hydrocarbons is well described in the prior art, whereby both acyclic and aromatic hydrocarbons are converted to correspondingly less saturated hydrocarbon products. One of the best known of these dehydrogenation processes is the conversion of alkyl aromatics, particularly ethylbenzene to styrene. In this process ethylbenzene is reacted at an elevated temperature over a dehydrogenation catalyst, such as iron oxide to form styrene. A process for the dehydrogenation of ethylbenzene to styrene and catalysts used for that reaction are disclosed in U.S. Patent No. 6,096,937. See also U.S. Patent No. 4,551,571.

The commercial process for the conversion of ethylbenzene to styrene is normally conducted in a series of radial, adiabatic reactors rather than in a single reactor. Radial reactors utilized for the production of styrene are disclosed in U.S. Patent Nos. 3, 475, 508, 3,515,763 and 3,918,918. These radial reactors generally are elongated, cylindrical, vertical structures which may be very large, ranging in diameter from 5 to 20 feet (150 to 610 cm) or more and in length from 5 to about 100 feet (150 to 3050 cm) or more. Examples of radial reactor designs are disclosed in the drawings associated with JP 49039971 and 49039972. A process and apparatus for the conversion of ethylbenzene to styrene in a radial reactor are also disclosed in U.S. Patent Nos. 5,358,698 and 4,039,601.

US 3,620,685 discloses a radial flow catalyst reactor which is used e.g. in the oxidative dehydrogenation of olefins to diolefins. Contained within the reactor are one or more, usually two, concentric layers or rings of catalyst. As an alternative other materials may be contained in the concentric layers. One layer can be catalyst, and the other layer or layers, if any, can be dead space filled with inert material. The feed stream is introduced into the reactor and then passes the catalyst layers from their outer side towards the center of the reactor.

It has been recognized that a reactor system containing multiple radial reactors may produce a higher degree of conversion of the hydrocarbon and may have greater product yield than is exhibited by use of a single radial reactor. Thus, sometimes three or more radial reactors are arranged in a serial flow orientation with reheat means, which may be located both within and between. the reactors, to add heat to the reaction.

Conventional radial reactors contain an inlet located in the center of the radial reactor assembly. Catalysts for the reaction are placed within a bed or beds in that reactor assembly, generally occupying a ring-shaped, vertical space, which is located outside of a central core of the reactor. The feed stream enters the reactor through the inlet and then flows radially outward through the catalyst material contained in the vertical catalyst bed to an open, annular space, which is formed outside of the catalyst bed but within the reactor assembly. Ultimately the feed stream flows to an outlet as shown, for example, in U.S. Patent No. 3,898,049.

Modifications to this basic design have been considered, such as are disclosed in U.S. Patent No. 5,358,698. The goal of such modified arrangements is to enhance certain performance and operating characteristics of the catalyst, such as selectivity and activity. "Selectivity" is the ability of a catalyst to selectively produce higher levels of the desired product and lower levels of undesired products. For example, in the conversion of ethylbenzene to styrene, higher selectivity results in higher levels of styrene and lower levels of various by-products. "Activity" is the ability of the catalyst to convert a certain percentage of the ethylbenzene during each pass of the feedstock over the catalyst. Higher activity means a higher percentage of the ethylbenzene is converted.

The conversion of ethylbenzene to styrene is an endothermic reaction which requires the addition of heat to the process to maintain an appropriate level of activity. Accordingly, the rate of flow of the ethylbenzene must be controlled as it passes across the catalyst bed to maintain acceptable selectivity and activity. Thus, the liquid hourly space velocity (LHSV) of the feed stream through the catalyst bed of the reactor must be regulated. The LHSV is generally maintained at a given rate regardless of the thickness of the catalyst bed in the reactor assembly.

Catalyst material loses selectivity and activity over time. Multiple reactors have been used to overcome this problem. One proposed design for such reactors is disclosed in U.S. Patent No. 6,096,937.

There are two entirely different recognized types of ethylbenzene dehydrogenation reaction. The conventional dehydrogenation reaction (nonoxidative dehydrogenation) occurs over an iron oxide catalyst and requires that oxygen not be present in the feed stream.

The less utilized, second type of dehydrogenation reaction is oxidative dehydrogenation where the feed stream contains ethylbenzene and a source of oxygen. In this second process, the feed stream is passed over an oxidative dehydrogenation catalyst, where an oxidation reaction occurs to generate heat. The reaction is generally as follows:

$$C_6H_5CH_2CH_3 + \tfrac{1}{2}O_2 \rightarrow C_6H_5CH = CH_2 + H_2O$$

Oxidative dehydrogenation is exothermic and irreversible. An oxidative dehydrogenation reaction is disclosed for example in U.S. Patent Nos. 5,510,553 and 4,777,319. This process is also discussed in Kirk-Othmer, *Encyclopedia of Chemical Technologies,* Volume 22, page 978 (1996).

In addition, UOP has disclosed a process for oxidatively reheating a reaction mixture including ethylbenzene using a radial reactor system containing two oxidation catalyst beds utilized in combination with three conventional dehydrogenation catalyst beds in U.S. Patent No. 5,043,500. In the process of this patent an oxidation catalyst bed (64) is physically located in a vertically layered bed next to a dehydrogenation catalyst (66) also contained within the reactor. UOP has disclosed the use of one, two or more oxidation catalyst beds. See also Kirk-Othmer, *Encyclopedia of Chemical Technologies*, Volume 22, pages 978-980 (1996).

Conventional "nonoxidative" dehydrogenation reactions generally use radial reactors but conventionally utilize only a single dehydrogenation catalyst, such as a conventional iron oxide catalyst containing a small amount of potassium and chrome as disclosed, for example, in U.S. Patent Nos. 2,866,790 and 2,866,791. Various catalysts for nonoxidative dehydrogenation are also disclosed in U.S. Patent No. 6,191,065, the contents of which are incorporated herein by reference.

Conventional nonoxidative dehydrogenation catalysts used for the conversion of ethylbenzene to styrene in reactors gradually deactivate during normal use, causing a reduction in ethylbenzene conversion. As part of the deactivation process, the catalyst loses potassium. During the dehydrogenation reaction, potassium migrates across the catalyst bed from the inlet side to the outlet side. Thus, the catalyst located closest to the inlet generally exhibits the greatest potassium loss over time.

Several attempts have been made to address the problem of loss of potassium from the dehydrogenation catalyst during a nonoxidative dehydrogenation reaction. For example, patents owned by Raytheon specifically teach methods and apparatus for regenerating and stabilizing dehydrogenation catalysts which have lost potassium during the dehydrogenation reaction. These patents include U.S. Patent Nos. 5,461,179, 5,686,369, 5,695,724 and 5,739,071. The preferred method disclosed by these patents comprises the continuous or intermittent introduction of an alkali metal or alkali metal compound to the reactant stream. For example, in one of these processes, a small amount of potassium hydroxide is vaporized continuously into the reaction feed stream. This potassium hydroxide is brought into contact with the catalyst. These patents report that catalyst activity and selectivity were improved by this addition. The process required the addition of from about 0.01 to about 100 parts per million of the alkali metal compound to the feed stream. Additional processes for the addition of the alkali metal compound include addition of the compound in the form of a dry solid powder and the use of a solid lump containing the alkali metal compound placed in the path of the heated reactant feed stream, which solid lump gradually vaporized during processing.

In radial reactors used for ethylbenzene dehydrogenation, the gas feed flows radially from the central core of the reactor assembly through catalyst material contained in a ring-shaped, vertical catalyst bed contained within the radial reactor. However, because many such catalytic reactions, including nonoxidative dehydrogenation of ethylbenzene, are temperature sensitive, the volume of the catalyst material within the catalyst bed that actually catalyzes the feed stream is often limited. For example, in nonoxidative dehydrogenation radial reactors for the dehydrogenation of ethylbenzene, only the first 4 inches (10 cm) to 15 inches (40 cm) or so of thickness of the catalyst material contained in the ring-shaped, vertical layer of the catalyst bed effectively dehydrogenates the ethylbenzene feed stream. This section of the catalyst bed also loses the greatest amount of the potassium during the dehydrogenation reaction. Because the reaction is adiabatic, by the time the ethylbenzene feed stream has passed 18 inches (46 cm) or so through the catalyst bed, the temperature of the feed stream has dropped to such an extent that the activity of the reaction is diminished dramatically or even extinguished. Further, when the temperature of the ethylbenzene feed stream drops as it passes through a thick catalyst bed, a higher percentage of undesired byproducts are produced. In addition, the greater the thickness of the catalyst bed, the greater the pressure drop as the feed stream passes through the catalyst bed.

Notwithstanding these reductions in the performance of the catalyst material in the thick catalyst beds contained in large diameter radial reactors, it has become conventional to build shorter reactor assemblies with thicker catalyst beds instead of building taller radial reactors with thinner catalyst beds because of the high cost in building both the support structure for the reactors and the radial reactors themselves. While shorter, thicker radial reactors contain the same overall quantity of catalyst material as taller, thinner radial reactors, the performance of these shorter, thicker reactors is not as efficient as when a taller, but narrower reactor is utilized.

Accordingly, it is an object of this invention to disclose a catalyst composition contained within a catalyst bed of a new or an existing radial reactor assembly which exhibits higher selectivity and/or activity than conventional catalyst compositions.

It is a further object of this invention to disclose a composition of catalyst material contained within a catalyst bed of a new or existing radial reactor assembly which results in a reduction in pressure drop over the pressure drop experienced when a conventional catalyst material is loaded in that same radial reactor.

It is a still further object of the invention to disclose a composition of catalyst material contained within a catalyst bed of a new or existing radial reactor assembly, which reduces the percentage of undesired byproducts that are produced in comparison with the byproducts produced by a conventional catalyst loading in that same catalyst bed.

It is a further object of this invention to disclose a loading of material within a catalyst bed utilized within a new or an existing radial reactor, wherein the material is loaded in an inner and an outer ring-shaped, vertical layer within the catalyst bed, wherein the inner, vertical layer is filled with a generally inert material, which may contain a potassium compound, and the outer, vertical layer is filled with an active catalyst material.

It is a further object of this invention to disclose a loading of material within a catalyst bed for a new or an existing radial reactor utilized for the nonoxidative dehydrogenation of an alkylaromatic feed stream, wherein the material is a combination of a dehydrogenation catalyst, placed within an outer, ring-shaped, vertical layer of the catalyst bed and a generally inert material, which may contain a potassium compound, placed within an inner, ring-shaped, vertical layer of the catalyst bed of the radial reactor.

It is another object of this invention to disclose a novel process for the nonoxidative dehydrogenation of an alkylaromatic feed stream in a new or an existing radial reactor which includes passing the alkylaromatic feed stream through a catalyst bed in the radial reactor, wherein the catalyst bed Contains a nonoxidative dehydrogenation catalyst placed within an outer, ring-shaped, vertical layer of the catalyst bed and a generally inert material, which may contain a potassium compound, placed within an inner, ring-shaped, vertical, layer of the catalyst bed.

These and other objects can be obtained by the disclosed radial reactor for catalytic reaction for gaseous or liquid feed streams according to claim 1 and a process for the non-oxidative dehydrogenation of an alkyl aromatic feed stream according to claim 9. Preferred embodiments of the radial reactor and the process are defined in the depending claims.

Summary of the invention

This invention is directed to a radial reactor for catalytic reaction for gaseous or liquid feed streams comprising:

- an inlet pipe for introducing said feed stream into the center of said radial reactor;
- a vertical annular catalyst bed comprising two or more ring-shaped, vertical layers of material;
- wherein an active catalyst material is placed in an outer ring-shaped, vertical layer of the catalyst bed and an inert material is contained within an inner ring-shaped, vertical layer of the catalyst bed, such that a feed stream introduced through the inlet pipe is radially distributed outwardly first through the inner ring-shaped, vertical layer and then through the outer ring-shaped, vertical layer.

In a preferred embodiment, the inert material comprises a material which neither promotes catalytic reactions nor interferes with the desired catalytic reaction that occurs within the radial reactor. Also preferably, the use of the inert material in the inner, ring-shaped, vertical layer, results in no additional pressure drop of the feed stream, and most preferably, it results in a reduced pressure drop of the feed stream in comparison with the pressure drop that occurs when the catalyst bed contains only active catalyst material. In a further preferred embodiment the inert material comprises an alpha alumina or ceramic material with a size similar to that of the active catalyst material or a monolithic structure. Different sized materials can be utilized as long as there is not significant movement of either the catalyst or the inert material within the catalyst bed after loading.

Preferably the thickness of the vertical layer that holds the catalyst material is from about 4 inches (10 cm) to about 36 inches (90 cm), more preferably from about 6 inches (15 cm) to about 24 inches (60 cm), most preferably about 18 inches (46 cm).

In an alternative preferred embodiment the potassium compound has been added to the inert material.

By "inert material with an added potassium compound" is meant a material that will not adversely impact the physical, chemical or performance characteristics of the catalyst material during reaction yet replace a portion or all of the potassium that is lost from the active catalyst material during normal catalytic activity of the active catalyst material.

The potassium compound is preferably selected from the group consisting from potassium oxide, potassium hydroxide, potassium carbonate and potassium bicarbonate. Further, the added potassium compounds preferably comprises at least 0.1 % of the inert material, by weight, preferably 1 % to 40 % of the inert material, by weight, and more preferably from 5 % to 20 % of the inert material, by weight.

Preferably the overall thickness of the ring-shaped, vertical layer of material contained within the radial reactor is at least 18 inches (45 cm), and preferably from 18 inches (45 cm) to 48 inches (120 cm).

According to a further embodiment the active catalyst material comprises a plurality of active catalyst products, at least two of which catalyst products have different performance characteristics.

This invention is also directed to a process for the non oxidative dehydrogenation of an alkyl aromatic feed stream wherein said alkyl aromatic feed stream is passing through a radial reactor comprising:

- an inlet pipe for introducing said feed stream into the center of said radial reactor;
- a vertical annular catalyst bed comprising two or more ring-shaped, vertical layers of material;
- wherein an active catalyst material is placed in an outer ring-shaped, vertical layer of the catalyst bed, and an inert material is contained within an inner ring-shaped, vertical layer of the catalyst bed, such that said feed stream introduced through the inlet pipe is radially distributed outwardly first through the inner ring-shaped, vertical layer and then through the outer ring-shaped, vertical layer.

Brief Drescription of the Drawing

Figure 1 is a schematic view of a radial reactor of the invention.
Figure 2 is a top view of the radial reactor of Figure 1.
Figure 3 is a cut-away perspective view of the catalyst bed of the radial reactor of Figure 1.

Detailed Description of the Invention

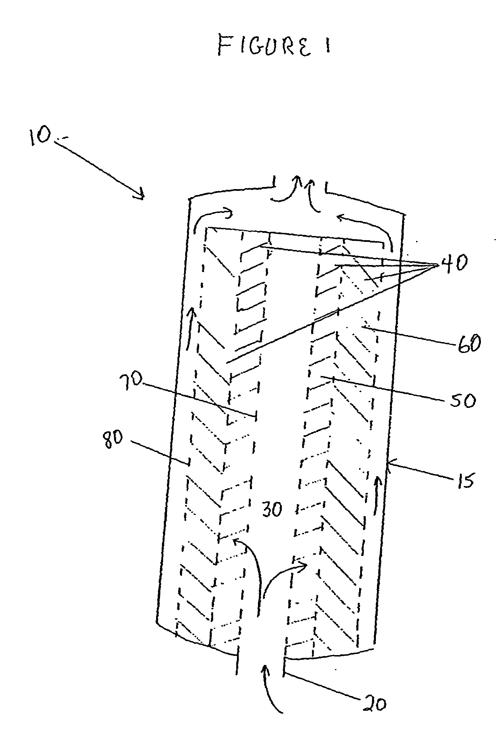

The invention is a composition for material loaded within a catalyst bed (40) within a radial reactor (10) utilized for catalytic reactions of gaseous or liquid feed streams, which reactor (10) includes a conventional radial reactor assembly (15), which contains a vertical, annular catalyst bed (40), wherein the catalyst bed is loaded with an active catalyst material (60), contained within a first ring-shaped layer of the catalyst bed (40), and a generally inert material (50) contained within a second ring-shaped layer of the catalyst bed (40). In an alternative embodiment a potassium compound is added to the inert material (50). Preferably, the first and second ring-shaped layers (50, 60) are in a vertical position in the radial reactor in relation to the facility in which the radial reactor is located as shown in Figure 1.

Referring to Figure 1, which is a cross-sectional schematic drawing showing one embodiment of the radial reactor (10) of the invention, a feed stream is introduced through an inlet pipe (20) into the center (30) of the radial reactor (10). When the reaction is the preferred nonoxidative dehydrogenation reaction, the hydrocarbon feed stream comprises an alkylaromatic material, preferably ethylbenzene. In this reaction the feed stream is heated by mixing it with super heated steam. The heated feed stream enters the inlet pipe (20) of the radial reactor (10) and is radially distributed through the catalyst bed (40) as shown by the arrows in Figure 1.

The catalyst bed (40) of the invention is comprised of two or more layers of material, preferably arranged in ring-shaped, vertical layers of material loaded in the reactor bed (40) as shown in Figures 1, 2 and 3. (While only a pair of vertical layers are shown in Figures 1, 2 and 3, three or more layers are within the scope of the invention. Other arrangements of the layers of material are also within the scope of the invention.) When two (2) layers of material are loaded within the catalyst bed (40), the two (2) layers preferably comprise a layer of an active catalyst material (60) and a layer of a generally inert material (50) which may contain a potassium compound. For example, when the reaction is the non-oxidative dehydrogenation of ethylbenzene, one layer of the catalyst bed is comprised of an active catalyst (60) for that reaction and the second layer is comprised of a generally inert material (50), which does not interfere with the nonoxidative dehydrogenation reaction and which may contain a potassium compound.

Each of the materials are preferably arranged in a separate vertical, annular layer within the radial reactor (10) as shown in Figure 1. Although formal separation of the two vertical layers of material can be effected by use of a device such as a screen, such as is shown in U.S. Patent No. 5,043,500, in a preferred embodiment the two separate layers of material (50, 60) are in intimate contact with each other within the radial reactor (10). In this preferred arrangement some mixing of the catalyst material with the generally inert material, which may contain a potassium compound, may occur at the boundary area between the different materials, although substantial mixing is not preferred.

The materials are retained in place within the catalyst bed (40) of the radial reactor (10) by use of an inner screen (70) and an outer screen (80), as shown in Figure 1.

In a preferred embodiment, the catalyst bed (40) contains at least two vertical layers (50, 60) of material, wherein at least one of the layers of material is the active catalyst material (60) and at least one of the layers is the inert material (50) onto which a potassium compound may be added. Many different combinations of active catalysts and generally inert materials, which may contain a potassium compound, are within the scope of the invention. For example, in a preferred embodiment for the non-oxidative dehydrogenation of or alkyl aromatic feed stream, a nonoxidative dehydrogenation catalyst, which has high activity is placed in the outer layer (60) of the catalyst bed (40) and a generally inert material, such as a ceramic or alpha alumina material onto which a potassium compound may be added, is placed within the inner layer (50) within the radial reactor (10).

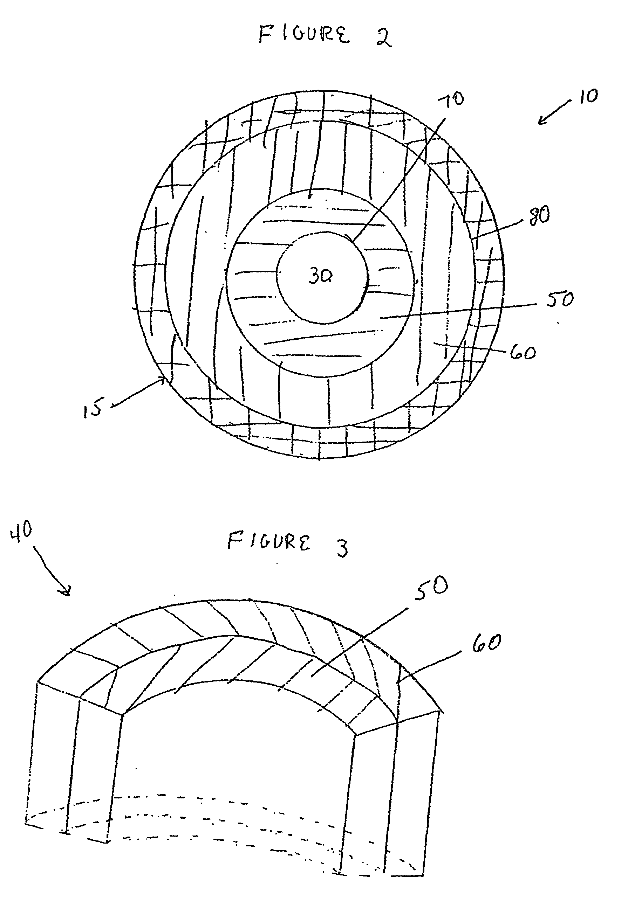

By placing the active catalyst material in the outer ring-shaped, vertical layer (60) of the catalyst bed (40), there is an increase in the overall initial surface area of the catalyst material that is exposed to the hot, preheated feed stream as the feed stream passes through the catalyst bed (40) as shown in Figure 3. There is an increase in the overall initial surface area of the catalyst layer because it is a further distance from the center (30) of the radial reactor (10) as shown in Figures 1, 2 and 3, thus presenting a greater vertical surface of catalyst material for reaction with the hot, preheated feed stream. Because the generally inert material, which may contain a potassium compound does not interfere with the reaction and because a greater surface area of the catalyst material is exposed to the feed stream, there is an increase in performance of the overall catalyst bed (40) over a conventional catalyst bed, wherein the inner ring-shaped, vertical layer (50) of the catalyst bed (40) is also filled with catalytic material.

This arrangement of material in a radial reactor (10) also results in a greater volume of catalytic material that is "effectively" utilized in the catalyst bed (40). In most reactions, the "effective" portion of the catalyst bed (40) that is utilized is only the first 4 inches (10 cm) to about 15 inches (40 cm) or so of the thickness of the catalyst bed (40). Because the design of the catalyst loading of the catalyst bed of the invention has shifted the effective volume of catalytic material further outward within the catalyst bed (40), as shown, for example, in Figure 3, there is a greater overall volume of catalyst material contained within the first 4 to 15 inches (10-40 cm) of the active catalyst material using the design of the catalyst bed of the invention than would be present in the first 4 to 15 inches (10-40 cm) of a catalyst bed that did not contain inert material, within an inner ring-shaped, vertical layer (50) of the catalyst bed (40).

There are other improvements which result from this combination of a generally inert material, which may contain a potassium compound, utilized with a catalytic material. For example, because the inert material, which may contain a potassium compound, preferably has greater physical stability than that of the catalytic material which it replaces, there is a reduction in both the initial pressure drop and the increase in pressure drop with aging of catalyst as the feed stream passes radially through the catalyst bed (40). The liquid hourly space velocity (LHSV) is also increased.

This arrangement also reduces unwanted by-products that are often produced by the reaction of catalytic material in a catalyst bed (40) which is very thick. For example, when a radial reactor (10) is utilized for the nonoxidative dehydrogenation of ethylbenzene to styrene, the temperature within the catalyst bed (40) drops dramatically as the feed stream passes radially through the catalyst material within the catalyst bed (40). Because other types of reactions are preferentially catalyzed by the catalyst material at lower temperatures, there is an increase in the amount of unwanted by-products that are produced the further the feed stream passes through the catalyst bed (40). By reducing the overall thickness of the active catalyst material within the catalyst bed (40) and by replacing the active catalyst material with the generally inert material, which may contain a potassium compound, there is a reduction in the overall by-products produced. This reduction in byproducts occurs because there is a reduction in the quantity of the catalyst material that is exposed to the feed stream after the main reaction has occurred. Because the generally inert material, which may contain a potassium compound, does not react with the components of the feed stream and because the radial reactor (10) is a closed system, there is no drop in the temperature of the feed stream during its passage through the inert material, which may contain a potassium compound. By reducing the overall thickness of the catalyst bed (40), there is less catalyst that is exposed to the feed stream at a lower temperature than with conventional catalyst beds, thus resulting in a reduction in unwanted byproducts.

If the alternative preferred embodiment is utilized, where the generally inert material contains a potassium containing compound, one of results that is achieved is a continuous addition of potassium to the catalytic material to replace partially or totally the potassium lost by the catalyst material during the non-oxidative dehydrogenation reaction. Conventional catalytic materials utilized for a dehydrogenation reaction contain from about 1 to about 20 percent potassium. During a non-oxidative catalytic dehydrogenation reaction, the highest temperature of reaction occurs near the edge of the catalytic material that is closest to the inlet (20) of the feed stream. Although selectivity and activity of the catalyst material is highest at this location, potassium contained in this catalytic material also begins to migrate away from this catalytic material quickest at this location, resulting in a reduction of the overall performance of the catalytic material.

In contrast, this arrangement of a generally inert material containing a potassium compound and a catalytic material of the invention permits the potassium contained within the generally inert material to migrate from that inert material to the catalytic material, replacing potassium which has been lost from the catalytic material during catalytic activity. This replacement of potassium allows the catalytic material to retain its catalytic activity for a period of time greater than is experienced by catalytic material without the addition of a potassium compound.

The thickness of the active catalyst material layer of the catalyst bed (40) of the invention may vary depending upon the type of reaction that is being catalyzed by the catalyst material. In one preferred embodiment when the catalyst material is utilized for the nonoxidative dehydrogenation of ethylbenzene to styrene, the effective thickness of the catalytic material is from about 4 inches (10 cm) to about 48 inches (120 cm), preferably from about 6 inches (15 cm) to about 36 inches (90 cm), most preferably from about 18 inches (45 cm) to about 24 inches (60 cm). When the thickness of the catalyst bed (40) in the radial reactor (10) is greater than the desired effective thickness of catalyst material for the catalytic reaction, the remaining space within the catalyst bed (40) is filled with the generally inert material to which a potassium compound may be added. The thickness of the ring-shaped, vertical layer (50) of the generally inert material, which may contain a potassium compound, thus varies depending upon the overall thickness of the catalyst bed (40) within the radial reactor (10) and depending on the type of reaction being catalyzed by the catalyst material. Conventional catalyst beds may vary in thickness from as thin as 18 inches (45 cm) or so to 4 feet (120 cm) or more. Thus, the amount of the generally inert material, which may contain a potassium compound, varies depending upon the amount of catalytic material necessary for an effective reaction. Regardless, any reduction in the amount of the catalyst to the optimum range of four (4) inches (10 cm) to about forty-eight (48) inches (120 cm) is helpful and results in an enhancement in the overall activity of the catalyst bed.

In one preferred embodiment for the dehydrogenation of ethylbenzene as shown in Figure 1, the dehydrogenation catalyst contained in the outer, ring-shaped, vertical layer (60) is any conventional commercial or proprietary dehydrogenation catalyst, such as Styromax® catalyst produced by Sud-Chemie Inc., which catalyst material is generally comprised of iron oxide and potassium oxide. In a particularly preferred embodiment the nonoxidative dehydrogenation catalyst material contained in the outer, ring-shaped, vertical layer (60) is selected from the catalysts disclosed in U.S. Patent Nos. 6,242,379, 6,191,065 and 6,177,602, which are incorporated herein by reference.

In a preferred embodiment, the nonoxidative dehydrogenation catalyst of this invention is composed of about 30 to about 90 weight percent iron oxide calcu-

lated as $Fe_2O_3$, about 1 to about 50 weight percent of the oxide, hydroxide, carbonate, or bicarbonate of an alkali metal, calculated as an oxide and less than about 1000 ppm of a noble metal, wherein the noble metal is preferably rhodium, platinum, palladium, ruthenium or iridium, wherein said weight percents are based on the total catalyst weight. More preferably the amount of the noble metal present is less than about 300 ppm. For palladium, rhenium and rhodium preferably the amount of metal is less than about 100 ppm, and most preferably less than 20 ppm. Preferably, the nonoxidative dehydrogenation catalyst also contains as promoters one or more of the following: about 0.5 to about 25 weight percent cerium oxide calculated as $CeO_2$, from about 0.5 to about 10.0 weight percent molybdenum oxide or tungsten oxide calculated as $MoO_3$ or $WO_3$, from about 0.2 to about 10.0 weight percent an alkaline earth metal oxide, preferably magnesium or calcium oxide. Additional components of the nonoxidative dehydrogenation catalyst may include from about 50 ppm to about 4.0 weight percent of chromium oxide calculated as $Cr_2O_3$ and from about 10 ppm to about 2000 ppm of titanium oxide calculated as $TiO_2$. The nonoxidative dehydrogenation catalyst may also include from about 0.1 to about 10.0 weight percent of the salt or oxide of one or more of the following: aluminum, silicon, zinc, manganese, cobalt, cadmium, vanadium and copper, alone or in combination, calculated on an elemental basis.

A particularly effective nonoxidative dehydrogenation catalyst contains from about 40 to about 90 weight percent iron oxide calculated as $Fe_2O_3$, from about 5 to about 20 weight percent of an alkali metal compound calculated as an alkali metal oxide and less than about 300 ppm of a noble metal, wherein the noble metal is preferably palladium, rhenium, rhodium, ruthenium, platinum or iridium. For palladium, rhenium and rhodium preferably the amount of metal present is less than 100 ppm, most preferably less than about 20 ppm. The source for the noble metal is selected from the group including elemental noble metals, preferably elemental palladium, elemental rhodium, elemental ruthenium, elemental platinum and elemental iridium, compounds containing noble metals, preferably compounds containing palladium and/or ruthenium and/or rhodium and/or platinum and/or iridium and combinations thereof. The nonoxidative dehydrogenation catalyst also preferably includes from about 0.5 to about 10.0 weight percent of a molybdenum or tungsten compounds calculated as $MoO_3$ or $WO_3$, and from about 4.0 to about 12.0 weight percent of a cerium compound, calculated as $CeO_2$, wherein all weight percents are based on the total weight of the nonoxidative dehydrogenation catalyst. Additional promoters may be included with this nonoxidative dehydrogenation catalyst as discussed above.

A most preferable nonoxidative dehydrogenation catalyst contains from about 40 to about 90 percent iron oxide calculated as $Fe_2O_3$, about 5 to about 20 percent of an alkali metal compound, preferably potassium oxide, about 4.0 to about 12 percent of cerium oxide calculated as $CeO_2$, about 0.5 to about 10.0 percent of molybdenum or tungsten oxide calculated as $MoO_3$ or $WO_3$, preferably molybdenum oxide, about 0.2 to about 10.0 percent of calcium or magnesium oxide, preferably calcium oxide, about 10 ppm to about 1000 ppm of titanium oxide calculated as $TiO_2$, about 100 ppm to about 2000 ppm of chromium oxide calculated as $Cr_2O_3$, and less than about 20 ppm of a noble metal, wherein the noble metal is preferably palladium, ruthenium, rhodium, platinum and/or iridium, and wherein the percentage is calculated on an elemental basis. Additional components that can be added to this catalyst include from about 0.1 to about 10.0 weight percent of an oxide of aluminum, silicon, manganese, copper, zinc, cadmium, vanadium, and cobalt, calculated on an elemental basis.

Two or more nonoxidative dehydrogenation catalysts may be utilized together within the active catalyst material layer of the radial reactor (10), each forming a different vertical layer as long as the overall thickness of the layer of the active catalyst material does not dramatically reduce the overall performance of the radial reactor (10). When more than one layer of nonoxidative dehydrogenation catalysts is utilized, preferably at least one of the catalysts has a different performance and/or operating characteristic than at least one of the other catalysts. Different layers of the same catalysts may also be sandwiched around a catalyst with different operating or performance characteristics, depending upon the overall performance or operating characteristics that are desired.

The generally inert material, which may contain a potassium compound, that is utilized within the catalyst bed (40) is preferably comprised of any material which does not adversely interfere with the catalytic reaction of the catalyst material contained in the catalyst bed (40). This generally inert material, which may contain a potassium compound, also should not react with the components of the feed stream to produce unwanted byproducts. Further, the generally inert material, which may contain a potassium compound, is preferably formed in a shape which limits the overall pressure drop through the catalyst bed (40). Further, the generally inert material, which may contain a potassium compound, should have adequate crush strength. The crush strength is preferably the same as or greater than that of the active catalyst material. The generally inert material, which may contain a potassium compound, is also preferably formed in a similar size and shape to the catalytic material for loading purposes, but can have larger or more numerous openings passing through the individual particles of the inert material, which may contain a potassium compound, to reduce the overall pressure drop.

When the reaction is a nonoxidative dehydrogenation reaction, the generally inert material is preferably an inert material with a surface area from about 0.1 to about 50 $m^2$/g, preferably from about 1 to about 20 $m^2$/g, such as an alpha alumina or a ceramic material including ceramic monoliths. If a potassium compound is to be added to the inert material, the generally inert material that is chosen should be one that can receive and adsorb an appropriate amount of the potassium compound.

If a potassium compound is to be added to the inert material, the amount of the potassium compound that is added to the inert material should be sufficient to replace substantially all potassium that is lost from the nonoxidative dehydrogenation catalyst during conventional processing of the alkylaromatic feed stream over the conventional life of the catalyst bed. The amount of the potassium compound that may be added to the inert base material depends on the composition of the generally inert material and may vary, sometimes dramatically. However, in a preferred embodiment, the amount of the potassium component that is added to the inert material should comprise, after addition, at least about 0.1 percent, preferably from about 1 to about 40 percent and most preferably from about 5 to about 20 percent of the inert material, by weight.

The potassium compounds that may be added to the inert base material include potassium oxide, potassium hydroxide, potassium carbonate and potassium carbonate or other similar potassium compounds and combination thereof. Potassium chloride should not be utilized as chloride ions may adversely affect the catalyst.

In one preferred embodiment of a nonoxidative dehydrogenation process of the invention as shown in Figures 1, 2 or 3, nonoxidative dehydrogenation catalysts are loaded into the outer, vertical layer (60) and generally inert materials, which may contain a potassium compound, are loaded in the inner, vertical layer (50) of the catalyst bed (40) of the radial reactor (10), forming separate, vertical layers within the radial reactor (10). In this preferred embodiment, the outer layer (60) of catalyst material is at least four (4) inches (10 cm) thick, preferably from about four (4) inches (10 cm) to about forty-eight (48) inches (120 cm) thick and most preferably from about six (6) inches (15 cm) to about thirty-six (36) inches (90 cm) thick. The remaining material (50) in the catalyst bed (40) is the generally inert material, which may contain a potassium compound, preferably an alpha alumina, ceramic material or a monolithic structure.

If the material includes a potassium compound, the potassium compound preferably is chosen from potassium oxide, hydroxide, carbonate or bicarbonate. It is not mandatory that all of the generally inert material contain a potassium compound. However, sufficient potassium compound should be added to the inert material so that it can replace a significant portion of the anticipated lost potassium material that was present in the active catalyst component. Because the generally inert material, which may contain a potassium compound, is placed closest to the source of heat, potassium compounds, if present, are encouraged to migrate from the inert material to the active catalyst material. Thus, an amount of potassium compound in excess of that required to replace all of the potassium material contained in the active catalyst material in the radial reactor may be helpful in assisting the retention of activity and selectivity of the dehydrogenation catalyst.

After the nonoxidative dehydrogenation catalyst and the generally inert material, which may contain the potassium compound, are loaded into the catalyst bed (40) of the radial reactor (10), the feed stream, preferably an alkylaromatic and steam, is then passed through the radial reactor (10).

By using this composition of materials for the catalyst bed (40), significant performance advantages are achieved in comparison to the performance of conventional radial reactors with catalyst beds with a thickness greater than about 18 to 48 inches (45-120 cm) which are loaded with only active catalyst material. Using the inventive design including the generally inert material, which may contain potassium compounds, the life of the catalytic material is lengthened and the overall selectivity and activity of the catalyst material is enhanced for an extended period of time. In addition, in the inventive design, the catalyst material in the outer ring-shaped, vertical layer (60) presents a higher surface area for reaction with the feed stream than if only catalyst material is utilized within the catalyst bed (40) because the overall surface area of the catalyst material portion of the catalyst bed (40) is greater the further one moves radially outward from the center (30) of the radial reactor (10) as shown in Figure 3. In addition, because the volume of the catalyst material which is exposed to the feed stream at the proper operating parameters is optimized, there is a greater "effective" utilization of the catalyst material, thus resulting in higher performance of the catalyst material within the catalyst bed (40). Further, by choosing the right size, shape and strength of the inert material, which may contain a potassium compound, there is no increase in pressure drop and preferably there is a reduction in the pressure drop as the feed stream passes through the catalyst bed (40). In addition, the effective LHSV is increased. Less unwanted byproducts are also produced because the catalyst material is more effectively utilized. Finally, depending upon the relative cost of the catalyst material versus the generally inert material, which may contain the potassium compound, there may be a reduction in the overall cost of a catalyst load within the catalyst bed of the radial reactor.

The principles, preferred embodiments and modes of the operation of the present invention have been described in the foregoing specification. The invention which is protected herein, however, is not to be construed as limited to the particular forms disclosed as these are to be regarded as illustrative rather than restrictive.

**Claims**

1. A radial reactor (10) for catalytic reaction for gaseous or liquid feed streams comprising:

   - an inlet pipe (20) for introducing said feed stream into the center of said radial reactor (10);
   - a vertical annular catalyst bed (40) comprising two or more ring-shaped, vertical layers of material;
   - wherein an active catalyst material is placed in an outer ring-shaped, vertical layer (60) of the catalyst bed (40) and an inert material is contained within an inner ring-shaped, vertical layer (50) of the catalyst bed (40), such that a feed stream introduced through the inlet pipe (20) is radially distributed outwardly first through the inner ring-shaped, vertical layer (50) and then through the outer ring-shaped, vertical layer (60).

2. The radial reactor of Claim 1 wherein a potassium compound has been added to the inert material (50).

3. The radial reactor of Claim 2 wherein the potassium compound is selected from the group consisting of potassium oxide, potassium hydroxide, potassium carbonate, and potassium bicarbonate.

4. The radial reactor of any of Claims 2 or 3 wherein the added potassium compound comprises at least 0.1 percent of the inert material (50), by weight, preferably 1 percent to 40 percent of the inert material, by weight, and more preferably from 5 percent to 20 percent of the inert material, by weight.

5. The radial reactor of any of Claims 1 to 4 wherein the layer containing the catalyst material (60) is at least 4 inches (10 cm) in thickness, preferably from 6 inches to 24 inches (15 cm to 60 cm) in thickness, when measured radially from a center of the radial reactor.

6. The radial reactor of any of Claims 1 to 5 wherein the inert material (50) comprises an alpha alumina or ceramic material with a size similar to that of the active catalyst material or a monolithic structure.

7. The radial reactor of any of Claims 1 to 6 wherein the overall thickness of the ring-shaped, vertical layer of material contained within the radial reactor is at least 18 inches (45 cm), and preferably from 18 inches (45 cm) to 48 inches (120 cm).

8. The radial reactor of any of Claims 1 to 7 wherein the active catalyst material (60) comprises a plurality of active catalyst products, at least two of which catalyst products have different performance characteristics.

9. A process for the non oxidative dehydrogenation of an alkyl aromatic feed stream wherein said alkyl aromatic feed stream is passing through a radial reactor (10) comprising:

   - an inlet pipe (20) for introducing said feed stream into the center of said radial reactor (10);
   - a vertical annular catalyst bed (40) comprising two or more ring-shaped, vertical layers of material;
   - wherein an active catalyst material is placed in an outer ring-shaped, vertical layer (60) of the catalyst bed, and an inert material is contained within an inner ring-shaped, vertical layer (50) of the catalyst bed, such that said feed stream introduced through the inlet pipe (20) is radially distributed outwardly first through the inner ring-shaped, vertical layer (50) and then through the outer ring-shaped, vertical layer.

10. The process of Claim 9 wherein a potassium compound has been added to the inert material (50).


**Patentansprüche**

1. Radialreaktor zur katalytischen Umsetzung von gasförmigen oder flüssigen Zufuhrströmen, umfassend:

   - ein Einlassrohr (20) zum Einführen des Zufuhrstroms in die Mitte des Radialreaktors (10);

   - ein vertikales, ringförmiges Katalysatorbett (40), umfassend zwei oder mehrere ringförmige, vertikale Materialschichten;

   - wobei ein aktives katalytisches Material in einer äußeren ringförmigen, vertikalen Schicht (60) des Katalysatorsbetts (40) angeordnet ist und ein inertes Material in einer inneren ringförmigen, vertikalen Schicht (50) des Katalysatorsbetts (40) angeordnet ist, so dass ein über das Einlassrohr (20) eingeführter Zufuhrstrom zuerst durch die innere ringförmige, vertikale Schicht (50) und anschließend durch die äußere ringförmige, vertikale Schicht (60) radial nach außen verteilt wird.

2. Radialreaktor nach Anspruch 1, wobei dem inerten Material (50) eine Kaliumverbindung zugesetzt ist.

3. Radialreaktor nach Anspruch 2, wobei die Kaliumverbindung ausgewählt ist aus der Gruppe, bestehend aus Kaliumoxid, Kaliumhydroxid, Kaliumcarbonat und Kaliumbicarbonat.

4. Radialreaktor nach einen der Ansprüche 2 oder 3, wobei die zugesetzte Kaliumverbindung einen Anteil an dem inerten Material (50) von mindestens 0,1 Gew.-%, vorzugsweise 1 bis 40 Gew.-% und insbesondere bevorzugt von 5 bis 20 Gew.-% an dem inerten Material hat.

5. Radialreaktor nach einem der Ansprüche 1 bis 4, wobei die das katalytische Material enthaltende Schicht (60) bei radialer Messung von einer Mitte des Radialrektors eine Dicke von mindestens 4 inch (10 cm), vorzugsweise von 6 inch bis 24 inch (15 cm bis 60 cm) aufweist.

6. Radialreaktor nach einem der Ansprüche 1 bis 5, wobei das inerte Material (50) ein $\alpha$-Aluminiumoxid oder ein keramisches Material umfasst, das eine vergleichbare Größe wie das aktive katalytische Material hat oder das eine monolithische Struktur aufweist.

7. Radialreaktor nach einem der Ansprüche 1 bis 6, wobei die Gesamtdicke der ringförmigen, vertikalen Schicht des im Radialreaktor enthaltenen Materials mindestens 18 inch (45 cm), vorzugsweise 18 inch (45 cm) bis 48 inch (120 cm) beträgt.

8. Radialreaktor nach einem der Ansprüche 1 bis 7, wobei das aktive katalytische Material (60) eine Vielzahl von aktiven katalytischen Produkten umfasst, wobei mindestens zwei der katalytischen Produkte unterschiedliche Leistungscharakteristika aufweisen.

9. Verfahren zur nichtoxidativen Dehydrierung eines Zufuhrstroms von Alkylaromaten, wobei der Zufuhrstrom der Alkylaromaten durch einen Radialreaktor (10) geleitet wird, der umfasst:

   - ein Einlassrohr (20) zur Einführung des Zufuhrstroms in die Mitte des Radialreaktors (10);

   - ein vertikales, ringförmiges Katalysatorbett (40), umfassend zwei oder mehrere ringförmige, vertikale Materialschichten;

   - wobei ein aktives katalytisches Material in einer äußeren ringförmigen, vertikale Schichtn (60) des Katalysatorsbettes angeordnet ist und ein inertes Material in einer inneren ringförmigen, vertikalen Schicht (50) des Katalysatorsbetts angeordnet ist, so dass ein über das Einlassrohr (20) eingeführter Zufuhrstrom zuerst durch die innere ringförmige, vertikale Schicht (50) und anschließend durch die äußere ringförmige, vertikale Schicht radial nach außen verteilt wird.

**10.** Verfahren nach Anspruch 9, wobei dem inerten Material (50) eine Kaliumverbindung zugesetzt ist.

**Revendications**

**1.** Réacteur radial (10) pour réaction catalytique pour courants d'alimentation gazeux ou liquides comprenant:

- un tuyau d'admission (20) pour introduire ledit courant d'alimentation dans le centre dudit réacteur radial (10);
- un lit catalytique annulaire vertical (40) comprenant deux couches en plus en forme d'anneau, verticales, de matériau ;
- dans lequel un matériau catalytiquement actif est placé dans une couche externe en forme d'anneau, verticale (60) du lit catalytique (40) et un matériau inerte est contenu à l'intérieur d'une couche interne, en forme d'anneau, verticale (50) du lit catalytique (40), de manière à ce qu'un courant d'alimentation introduit par le tuyau d'admission (20) soit radialement réparti vers l'extérieur d'abord au travers de la couche interne, en forme d'anneau, verticale (50) et ensuite au travers par la couche externe, en forme d'anneau, verticale (60).

**2.** Réacteur radial selon la revendication 1, dans lequel un composé du potassium a été ajouté au matériau inerte (50).

**3.** Réacteur radial selon la revendication 2, dans lequel le composé du potassium est choisi dans le groupe constitué par l'oxyde de potassium, l'hydroxyde de potassium, le carbonate de potassium, et le bicarbonate de potassium.

**4.** Réacteur radial selon l'une quelconque des revendications 2 ou 3, dans lequel le composé du potassium ajouté comprend au moins 0,1 pour cent du matériau inerte (50), en poids, de préférence 1 pour cent à 40 pour cent du matériau inerte, en poids, et de préférence encore de 5 pour cent à 20 pour cent du matériau inerte, en poids.

**5.** Réacteur radial selon l'une quelconque des revendications 1 à 4, dans lequel la couche contenant le matériau catalytique (60) a une épaisseur d'au moins 4 inches (10 cm), de préférence une épaisseur de 6 inches à 24 inches (15 cm à 60 cm), lorsqu'elle est mesurée radialement à partir d'un centre du réacteur radial.

**6.** Réacteur radial selon l'une quelconque des revendications 1 à 5, dans lequel le matériau inerte (50) comprend un matériau d'alpha alumine ou de céramique avec une taille similaire à celle du matériau catalytiquement actif ou une structure monolithique.

**7.** Réacteur radial de l'une quelconque des revendications 1 à 6, dans lequel l'épaisseur totale de la couche en forme d'anneau, verticale, de matériau contenue à l'intérieur du réacteur radial est d'au moins 18 inches (45 cm), et de préférence de 18 inches (45 cm) à 48 inches (120 cm).

**8.** Réacteur radial de l'une quelconque des revendications 1 à 7, dans lequel le matériau catalytiquement actif (60) comprend une pluralité de produits catalytiquement actifs, au moins deux parmi ces produits catalytiquement actifs ayant des caractéristiques de fonctionnement différentes.

**9.** Procédé pour la déshydrogénation non oxydative d'un courant d'alimentation alkyl aromatique, dans lequel ledit courant d'alimentation alkyl aromatique passe à travers un réacteur radial (10) comprenant:

- un tuyau d'admission (20) pour introduire ledit courant d'alimentation dans le centre dudit réacteur radial (10);
- un lit catalytique annulaire vertical (40) comprenant au moins deux couches en forme d'anneau, verticales, de matériau ;
- dans lequel un matériau catalytiquement actif est placé dans une couche externe en forme d'anneau, verticale (60) du lit catalytique (40) et un matériau inerte est contenu à l'intérieur d'une couche interne, en forme d'anneau, verticale (50) du lit catalytique, de manière à ce que ledit courant d'alimentation introduit par le tuyau d'admission (20) soit radialement réparti vers l'extérieur d'abord au travers de la couche interne, en forme d'anneau, verticale (50) et ensuite au travers de la couche externe, en forme d'anneau, verticale (60).

**10.** Procédé de la revendication 9, dans lequel un composé du potassium a été ajouté au matériau inerte (50).

FIGURE 1

FIGURE 2

FIGURE 3